# EUROPÄISCHE PATENTANMELDUNG

(11) **EP 3 064 245 A1**
(43) Veröffentlichungstag der Anmeldung: **07.09.2016**
(21) Anmeldenummer: 16158510.4
(22) Anmeldetag: 03.03.2016
(51) Int. Cl.: A61M 25/09

(54) **FÜHRUNGSDRAHT ZUM FÜHREN EINES GEGENSTANDES ZU EINEM ZIELORT IN EINER HOHLSTRUKTUR EINES MENSCHLICHEN ODER TIERISCHEN KÖRPERS**

(30) Priorität: 06.03.2015 DE 102015204065
(71) Anmelder: Otto-von-Guericke-Universität Magdeburg, 39120 Magdeburg (DE)
(72) Erfinder: Weigt, Jochen, Dr., 39120 Magdeburg (DE)
(74) Vertreter: Kailuweit & Uhlemann Patentanwälte Partnerschaft mbB

(57) **Zusammenfassung**

Die Erfindung betrifft Führungsdrähte zum Führen eines Gegenstandes zu einem Zielort in einer Hohlstruktur eines menschlichen oder tierischen Körpers.

Die Führungsdrähte zeichnen sich insbesondere dadurch aus, dass mittels des Führungsdrahtes eine Kanülierung/Sondierung von kleinen Hohlstrukturen in menschlichen und tierischen Körpern einfach durchführbar ist.

Dazu weist der Endenbereich des Führungsdrahts in Richtung zu dessen Ende eine erste Abwinkelung und beabstandet zur ersten Biegung mindestens eine zweite Abwinkelung auf. Die Abwinkelungen liegen dabei räumlich nicht in derselben Ebene. Weiterhin ist der Bogen so ausgebildet, dass das Ende des Führungsdrahtes in Richtung der verlängerten Symmetrieachse des Führungsdrahtes weist, in Richtung weg von der verlängerten Symmetrieachse des Führungsdrahtes weist oder in der verlängerten Symmetrieachse angeordnet ist.

Der Führungsdraht ist damit ein mehrfach gebogener/geknickter Führungsdraht. Diese werden als medizinische Führungsdrähte bei einer Vielzahl von Verfahren zum Führen von Kathetern oder anderen Vorrichtungen zu Zielorten innerhalb des Körpers eines Patienten eingesetzt.

## Beschreibung

Die Erfindung betrifft Führungsdrähte zum Führen eines Gegenstandes zu einem Zielort in einer Hohlstruktur eines menschlichen oder tierischen Körpers.

Führungsdrähte mit einem Kerndraht und einem distalen Wendelende sind unter anderem in den Druckschriften US 5 365 942 A, US 4 964 409 A, US 4 846 186 A, US 4 748 986 A, US 4 554 929 A und US 4 545 390 A beschrieben. Aus diesen beschreiben die Druckschriften darüber hinaus US 5 365 942 A, US 4 846 186 A und US 4 554 929 A jeweils Wendelenden mit beabstandeten Windungen.

Die Druckschriften US 5 454 373 A, US 5 443 455 A, US 5 416 131 A, US 5 242 428 A, US 5 217 026 A, US 5 135 516 A, WO 1993 010 827 A1, WO 1991 019 756 A1, EP 661 072 A1 und EP 591 061 A1 beinhalten Katheter und Führungsdrähte mit hydrophilen Beschichtungen.

In Kunststoffhüllen eingeschlossene Führungsdrähte sind unter anderem aus den Druckschriften US 5 452 726 A, US 5 333 620 A und EP 661 073 A1 bekannt.

Verfahren und Materialien zur hydrophilen Beschichtung medizinischer und anderer Vorrichtungen sind Inhalt der Druckschriften US 5 331 027 A, US 5 069 899 A, US 5 037 677 A, US 5 001 009 A, US 4 959 074 A, US 4 801 475 A und US 4 663 233 A.

Dabei bestehen solche Führungsdrähte aus einem aus Edelstahl, Nickeltitanlegierungen, Tantal oder anderen Metallen gebildeten Kerndraht. Das Ende ist typischerweise ein wendelförmig gewickeltes Filament aus einem verformbaren Metall, das von dem Arzt zur Erleichterung der Platzierung verformt werden kann oder bereits eine bestimmte Form aufweist. Letzterer ist dabei mehrfach in verschiedenen Ebenen gebogen. Damit soll das Erreichen bestimmter anatomischer Strukturen erleichtert werden, wobei weiterhin durch die erhöhte Flexibilität unbeabsichtigte Verletzungen vermieden werden sollen. Zur leichteren Sondierung wird das aus dem Körper oder aus der medizinischen Vorrichtung ragende Ende des Drahtes mit einer Vorrichtung rotiert und dabei vor und zurück geschoben, bis die Stenose oder der Verschluss überwunden wurde.

Die Druckschrift US 8 852 204 B2 beinhaltet eine Schlinge an einem Draht. Damit können verschiedene andere Instrumente an dem Draht fixiert werden, so dass verschiedene Handlungen im Körper durchführbar sind. Das können Ablationen, Bergen von Fremdkörpern oder ähnliches sein. Der Draht weist in lediglich einer räumlichen Ebene einen Bogen auf.

Die Druckschrift US 4 554 929 A offenbart einen ausgezogenen Draht mit einer Spiralummantelung.

Durch die Druckschrift US 4 846 186 A ist ein gebogener Draht mit Spiralummantelung bekannt. Dieser ist nur in einer Richtung und damit in einer Ebene gebogen. Das ist aus dem gezeigten Querschnitt ableitbar. Der hier gezeigte Draht ist beidseitig abgeflacht.

Die Druckschrift US 5 443 455 A beinhaltet eine Art der Herstellung eines Drahtes. Das betrifft insbesondere eine spezielle Metalloberflächenbehandlung von Drähten. Der Draht zeichnet sich dabei durch seine Biegsamkeit aus. Im Normalzustand ist dieser nicht gebogen.

Der im Patentanspruch 1 angegebenen Erfindung liegt die Aufgabe zugrunde, dass mittels des Führungsdrahtes eine Kanülierung/Sondierung von kleinen Hohlstrukturen in menschlichen und tierischen Körpern einfach durchführbar ist.

Diese Aufgabe wird mit den im Patentanspruch 1 aufgeführten Merkmalen gelöst.

Die Führungsdrähte zum Führen eines Gegenstandes zu einem Zielort in einer Hohlstruktur eines menschlichen oder tierischen Körpers zeichnen sich insbesondere dadurch aus, dass mittels des Führungsdrahtes eine Kanülierung/Sondierung von kleinen Hohlstrukturen in menschlichen und tierischen Körpern einfach durchführbar ist.

Dazu weist der Endenbereich des Führungsdrahts in Richtung zu dessen Ende eine erste Abwinkelung und beabstandet zur ersten Biegung mindestens eine zweite Abwinkelung auf. Die Abwinkelungen liegen dabei räumlich nicht in derselben Ebene. Weiterhin ist der Bogen so ausgebildet, dass das Ende des Führungsdrahtes in Richtung der verlängerten Symmetrieachse des Führungsdrahtes weist, in Richtung weg von der verlängerten Symmetrieachse des Führungsdrahtes weist oder in der verlängerten Symmetrieachse angeordnet ist.

Der Führungsdraht ist damit ein mehrfach gebogener/geknickter Führungsdraht. Diese werden als medizinische Führungsdrähte bei einer Vielzahl von Verfahren zum Führen von Kathetern oder anderen Vorrichtungen zu Zielorten innerhalb des Körpers eines Patienten eingesetzt. Der Führungsdraht dient der Kanülierung/Sondierung von kleinen Hohlstrukturen in menschlichen aber auch tierischen Körpern, insbesondere von Gallengängen, Gallenblase dem Bauchspeicheldrüsengang sowie von Gefäßen.

Insbesondere ist der Führungsdraht zur Kanülierung von Engstellen (Stenosen) und Verschlüssen aber auch zur gezielten Sondierung von nicht stenotischen Anteilen geeignet. Von besonderem Interesse ist dabei die Applikation des Führungsdrahtes über eine medizinische Vorrichtung zur Führung eines Katheters oder einer Schleuse und zur Führung von Interventionen am entsprechend untersuchten Organ.

Dabei kann der Führungsdraht durch Rotation an der Längsachse und durch die Abwinkelungen leichter und gezielter in den anatonischen Strukturen manövriert werden. Gleichzeitig können Engstellen gezielter überwunden werden. Das wird dadurch erreicht, dass auf Grund der räumlichen Konfiguration der Drahtspitze als Endenbereich des Führungsdrahts kann diese besser mit der Achse der zu sondierenden anatonischen Struktur/Striktur in Deckung gebracht werden.

Die Abstände der Abwinkelungen als Biegungen oder Knicke sind variabel ausgestaltbar. Durch eine Änderung der Steigung und/oder der Abstände ändert sich die räumliche Konfiguration der Drahtspitze als Endenbereich des Führungsdrahts. Damit ist ein entsprechend der vorhandenen anatomischen Strukturen anpassbarer Führungsdraht gegeben.

Vorteilhafte Ausgestaltungen der Erfindung sind in den Patentansprüchen 2 bis 11 angegeben.

Der Bogen ist nach der Weiterbildung des Patentanspruchs 2 der Endenbereich einer linksgängigen oder rechtsgängigen konischen Raumspirale.

Der Bogen ist nach der Weiterbildung des Patentanspruchs 3 der Endenbereich einer linksgängigen oder rechtsgängigen Helix.

Die Steigung der konischen Raumspirale oder der Helix ist nach der Weiterbildung des Patentanspruchs 4 konstant oder nicht konstant.

Die Steigung der konischen Raumspirale oder der Helix nimmt nach der Weiterbildung des Patentanspruchs 5 in Richtung des Endes des Führungsdrahtes zu.

Die Anzahl der Windungen der Raumspirale oder der Helix ist nach der Weiterbildung des Patentanspruchs 6 gleich/größer 0,25.

Die Abwinkelung ist nach der Weiterbildung des Patentanspruchs 7 vorteilhafterweise ein Bogen oder ein Knick.

Nach der Weiterbildung des Patentanspruchs 8 bestimmen die Abstände der Abwinkelungen und deren Winkel zueinander die räumliche Anordnung des Endenbereichs des Führungsdrahtes.

Der Endenbereich des Führungsdrahtes ist nach der Weiterbildung des Patentanschpruchs 9 wenigstens ein Bogen.

Der Führungsdraht besteht nach der Weiterbildung des Patentanspruchs 10 aus einem Stahl oder Nitinol. Weiterhin weist der Führungsdraht eine hydrophile Kunststoffbeschichtung oder Polymerbeschichtung auf.

Günstigerweise besteht die Schicht nach der Weiterbildung des Patentanspruchs 11 aus Polyurethan, Polytetrafluorethylen, Silikon, einem Polyolefin, beispielsweise Polyovinylpyrrolidon, und/oder einem Polysaccharid, beispielsweise Hyaluronsäure oder Chondroitinsulfat.

Ein Ausführungsbeispiel der Erfindung ist in der Zeichnung prinzipiell dargestellt und wird im Folgenden näher beschrieben.

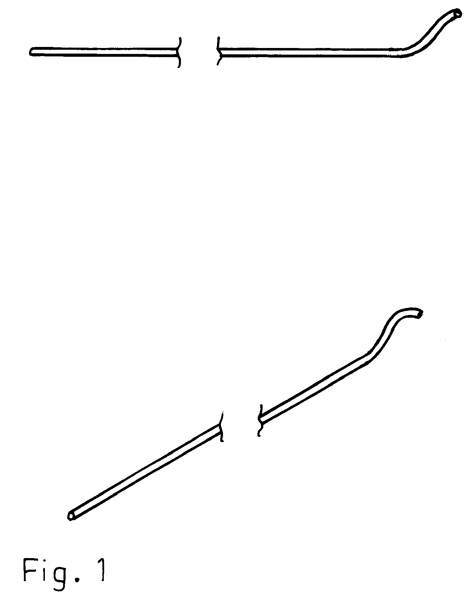

Es zeigt
Fig. 1 einen Führungsdraht zum Führen eines Gegenstandes zu einem Zielort in einer Hohlstruktur eines menschlichen oder tierischen Körpers in zwei Darstellungen.

Ein Führungsdraht zum Führen eines Gegenstandes zu einem Zielort in einer Hohlstruktur eines menschlichen oder tierischen Körpers weist im Endenbereich in Richtung zu dessen Ende eine erste Abwinkelung als Biegung und beabstandet zur ersten Biegung mindestens eine zweite Abwinkelung als Biegung auf.

Die Fig. 1 zeigt einen Führungsdraht zum Führen eines Gegenstandes zu einem Zielort in einer Hohlstruktur eines menschlichen oder tierischen Körpers in zwei prinzipiellen Darstellungen.

Der Endenbereich des Führungsdrahts besitzt die erste Biegung und beabstandet zu dieser die mindestens eine zweite Biegung, so dass der Endenbereich des Führungsdrahtes wenigstens ein Bogen ist.

Beispielsweise besitzt der Führungsdraht eine Länge jeweils einschließlich 100 mm bis 4.500 mm bei einem Durchmesser von bis 0,4 mm bis 1 mm. Weiterhin ist die erste Biegung 6 mm bis 12 mm und die zweite Biegung 3 mm bis 8 mm von der Spitze des Führungsdrahtes entfernt.

Der Bogen ist eine linksgängige oder rechtsgängige konische Raumspirale oder Helix. Die Steigung der konischen Raumspirale oder der Helix ist konstant oder nicht konstant. Bei der letzteren Variante kann die Steigung der konischen Raumspirale oder der Helix in Richtung des Endes des Führungsdrahtes zunehmen.

Das Ende des Führungsdrahtes
- weist in Richtung der verlängerten Symmetrieachse des Führungsdrahtes,
- weist in Richtung weg von der verlängerten Symmetrieachse des Führungsdrahtes oder
- ist in der verlängerten Symmetrieachse angeordnet.

Die Anzahl der Windungen der Raumspirale oder der Helix kann gleich/größer 0,25 sein.

Der Führungsdraht besteht aus einem Stahl oder Nitinol und weist eine hydrophile Kunststoffbeschichtung oder Polymerbeschichtung auf. Die Schicht kann dazu aus PTFE, Silikon, einem Polyolefin, beispielsweise Polyovinylpyrrolidon, und/oder einem Polysaccharid, beispielsweise Hyaluronsäure oder Chondroitinsulfat, bestehen.

## Patentansprüche

1. Führungsdraht zum Führen eines Gegenstandes zu einem Zielort in einer Hohlstruktur eines menschlichen oder tierischen Körpers, **dadurch gekennzeichnet, dass** der Endenbereich des Führungsdrahts in Richtung zu dessen Ende eine erste Abwinkelung und beabstandet zur ersten Biegung mindestens eine zweite Abwinkelung aufweist, so dass die Abwinkelungen räumlich nicht in derselben Ebene liegen und das Ende des Führungsdrahtes in Richtung der verlängerten Symmetrieachse des Führungsdrahtes weist oder das Ende des Führungsdrahtes in Richtung weg von der verlängerten Symmetrieachse des Führungsdrahtes weist oder das Ende des Führungsdrahtes in der verlängerten Symmetrieachse angeordnet ist.

2. Führungsdraht nach Patentanspruch 1, **dadurch gekennzeichnet, dass** der Bogen der Endenbereich einer linksgängigen oder rechtsgängigen konischen Raumspirale ist.

3. Führungsdraht nach Patentanspruch 1, **dadurch gekennzeichnet, dass** der Bogen der Endenbereich einer linksgängigen oder rechtsgängigen Helix ist.

4. Führungsdraht nach Patentanspruch 2 oder 3, **dadurch gekennzeichnet, dass** die Steigung der konischen Raumspirale oder der Helix konstant oder nicht konstant ist.

5. Führungsdraht nach Patentanspruch 4, **dadurch gekennzeichnet, dass** die Steigung der konischen Raumspirale oder der Helix in Richtung des Endes des Führungsdrahtes zunimmt.

6. Führungsdraht nach Patentanspruch 2 oder 3, **dadurch gekennzeichnet, dass** die Anzahl der Windungen der Raumspirale oder der Helix gleich/größer 0,25 ist.

7. Führungsdraht nach Patentanspruch 1, **dadurch gekennzeichnet, dass** die Abwinkelung ein Bogen oder ein Knick ist.

8. Führungsdraht nach Patentanspruch 1, **dadurch gekennzeichnet, dass** die Abstände der Abwinkelungen und deren Winkel zueinander die räumliche Anordnung des Endenbereichs des Führungsdrahtes bestimmen.

9. Führungsdraht nach Patentanspruch 1, **dadurch gekennzeichnet, dass** der Endenbereich des Führungsdrahtes wenigstens ein Bogen ist.

10. Führungsdraht nach Patentanspruch 1, **dadurch gekennzeichnet, dass** der Führungsdraht aus einem Stahl oder Nitinol besteht und dass der Führungsdraht eine hydrophile Kunststoffbeschichtung oder Polymerbeschichtung aufweist.

11. Führungsdraht nach Patentanspruch 10, **dadurch gekennzeichnet, dass** die Schicht aus Polyurethan, Polytetrafluorethylen, Silikon, einem Polyolefin und/oder einem Polysaccharid besteht.
